# EUROPEAN PATENT APPLICATION

(11) **EP 2 386 295 A2**
(43) Date of publication of application: **16.11.2011**
(21) Application number: 11156296.3
(22) Date of filing: 09.09.2005
(51) Int. Cl.: A61K 9/28, A61K 31/573

(54) **Tablets with site time-controlled gastrointestinal release of active ingredients**

(30) Priority: 10.09.2004 DE 102004043863; 01.09.2005 US 216469
(62) Divisional of application: 05778455.5
(71) Applicant: JAGOTEC AG, 4132 Muttenz (CH)
(72) Inventor: Schäffler, Achim, 64743, Beerfeden (DE); Vergnault, Guy, F-68680, Loechle (FR); Grenier, Pascal, F-68510, Keppelen (FR)
(74) Representative: Bevan, Emma

(57) **Abstract**

The present invention describes a pharmaceutical dosage form with site and time-controlled gastrointestinal release of a glucocorticoid.

## Description

The present invention describes a pharmaceutical dosage form with site- and time-controlled gastrointestinal release of a glucocorticoid.

Release of non-steroidal anti-inflammatory drugs in the stomach frequently causes ulcers of the gastric mucosa. This is why tablets with a gastro-resistant coating are now employed almost exclusively. The disadvantage is often that the active ingredient is released very quickly on entry into the intestine. Thus, it is possible with this technology to achieve control only of the site, but not of the timing, of active ingredient release.

Absorption of some active ingredients is possible only in certain sections of the gastrointestinal tract (absorption window). Active ingredient entry/transfer into the plasma is often desired only when the pathological state becomes particularly manifest at certain periods of the day (circadian rhythm). This is the case for example with asthma or ischemias in the early morning, joint pain in the morning, etc. On the other hand, the effect of some medicaments is often desired only locally in the gastrointestinal tract, such as for inflammations (e.g. in ulcerative colitis or Crohn's disease) or infections in the gastrointestinal tract.

Coated tablets have been described frequently, especially with the aim of delayed release of an active ingredient, in which case an initial phase without release of the active ingredient (lag phase) is followed by the active ingredient leaving the tablet.

Thus, WO 02/072033 discloses that the amount of coating material applied determines the lag phase. The coating consists of a swellable material through the pores of which the active ingredient is then released. In this case, the diffusion through the swellable matrix of the coating becomes the release-determining factor. However, release through the pores often does not take place spontaneously after the desired lag phase; on the contrary, there is onset of more or less rapid release. In addition, the influences of food on swelling and eroding coatings are very important.

US 5 464 633 describes a tablet for delayed release of an active substance. The tablet consists of a core which comprises the active ingredient and a polymer, and of a polymer-containing coating.

EP 0 463 877 describes a pharmaceutical preparation for controlled release of an active ingredient, which comprises a core and a coating layer, where the coating layer comprises a water-repellent salt and a copolymer.

A pharmaceutical preparation consisting of a core and of a multilayer coating for release of the active ingredient in the lower part of the gastrointestinal tract (colon) is known for example from EP 0 366 621. Film coatings which are degraded only in the colon by bacteria present therein are, however, unsuitable for releasing the active ingredient in upper sections of the intestine.

WO 01/80824 (Eurand) describes a pharmaceutical form having a core which, besides the active ingredient, also comprises a hydrop hilic, swelling polymer, and having a surrounding coating consisting of at least one water-insoluble polymer.

EP 0 939 623 B1 and US 6 183 780 (Duphar) describe an oral dosage form with delayed release consisting of a core and of a coating, where the coating consists of one or more polymers, of a water-soluble plasticizer and of a substance which increases the brittleness of the coating. The disadvantages of this form are, in particular, that influences of food are possible.

EP 1 067 910 (Bar-Shalom) describes an oral dosage form having at least one erodable surface. EP 1 275 381 (Yamanouchi) likewise describes a core tablet with coating, the latter consisting of a swellable hydrophilic polymer. The effects of food in these cases are also great.

Administration of dilitiazem in the form of biologically inert pellets with a plurality of layers is described in US 6 620 439 (Elite Labs). In this case, the active ingredient is released some hours after intake to treat arterial occlusions in the morning.

US Patent 5 792 476 describes a pharmaceutical composition for peroral administration for rheumatoid arthritis, which comprises a glucocorticoid as active ingredient and which leads to release in the small intestine. The composition is a granulate which is laminated with an inner layer which is resistant to a pH of 6.8, and with an outer layer which is resistant to a pH of 1.0.

US Patent 6 488 960 describes a pharmaceutical dosage form for controlled release of corticoids, reference being made to the formulations described in US Patent 5 792 476.

WO 01/08421 describes a tablet having a core which is coated by at least two layers, one of which completely encloses the other. The coating layers can be produced by spray coating and/or pressing.

WO 01/68056 discloses a pharmaceutical preparation having a release profile with a time delay, comprising a core and at least one hydrophilic or lipophilic coating surrounding the core, where the coating is slowly swollen, dissolved, eroded or changed in its structure in another way through the water present in the release medium, so that the core or parts of the core become accessible to the release medium. The coating may be formed for example as pressed coating.

WO 02/072034 discloses a pharmaceutical dosage form for delayed release, having a core which comprises as active ingredient a glucocorticoid and a material which brings about delayed release and includes at least one natural or synthetic gum.

WO 2004/093843, the content of which is incorporated herein by reference, discloses a tablet with a specific core geometry to release the active ingredient in a specific delayed release manner.

The problem underlying the present invention was to provide a pharmaceutical dosage form with site- and time-controlled release of a glucocorticoid, which makes reproducible *in vivo* release possible in the particular desired sections of the intestine irrespective of the patient's food intake. It was further intended also for the glucocorticoid release process itself to be controllable as optimally as possible depending on the relevant medical indication.

This problem is solved by a pharmaceutical dosage form with site- and time-controlled gastrointestinal release of a glucocorticoid, comprising
(a) a core having at least one glucocorticoid and having at least one swellable adjuvant such that the glucocorticoid is rapidly released from the dosage form when the core is contacted with gastrointestinal fluids; and
(b) an inert coating pressed onto the core, said coating being capable of preventing substantial release of the glucocorticoid for a defined time period following ingestion of the dosage form.

In another aspect, the present invention is directed to a method for the treatment of a patient in need of therapy with a glucocorticoid in a site- and time-controlled dosage form, said method comprising administering to said patient the pharmaceutical dosage form described herein.

In another aspect, the present invention is directed to a kit comprising at least one unit dosage of a dosage form described herein with site- and time- controlled gastrointestinal release of a glucocorticoid. The kit optionally contains instructional material for use of the unit dosage form.

In one aspect, the present invention relates to a method of producing a tablet which releases a glucocorticoid active ingredient at a predetermined variable location in the Gl tract, said method comprising:
determining the location in the Gl tract at which it is desired to deliver the glucocorticoid;
forming a coated tablet having a core comprising the glucocorticoid and a swellable adjuvant, and an inert outer coating; and
compressing the coating of said tablet at a pressure chosen to result in the release of the glucocorticoid at said predetermined position.

In another aspect, the present invention relates to a coated tablet having a core of a glucocorticoid active ingredient and a coating, capable of releasing the glucocorticoid at a predetermined variable location the Gl tract, the coating being compressed to a degree which results in the release of the glucocorticoid at said predetermined location.

In another aspect, the present invention relates to a method of producing a tablet which releases a glucocorticoid active ingredient at a predetermined variable location in the Gl tract, said method comprising:
determining the location in the Gl tract at which it is desired to deliver the glucocorticoid;
forming a coated tablet having a core comprising the glucocorticoid and a swellable adjuvant, and an inert outer coating;
compressing the coating of said tablet at a pressure chosen to result in the release of the glucocorticoid at said predetermined position; and
testing the *in vitro* release characteristics in a dissolution apparatus in order to confirm release of the glucocorticoid at a specific lag time.

In another aspect, the present invention relates to a method for the treatment of a local bowel disorder in the lower sections of the intestine, which comprises administering to a patient in need thereof a coated tablet having a core of a glucocorticoid active ingredient and a coating, the coating being compressed to a degree that results in the release of the glucocorticoid in the lower sections of the intestine.

### Drawings

**Figure 1** shows *the in vitro* release of the novel tablet containing 5 mg of prednisone ("Prednisone TR") with a lag phase of about 4 h (500 ml of water, paddle, USP)
**Figure 2** shows *the in vivo* plasma level of prednisone after administration of
   A) standard "Prednisone IR" (=immediate release) tablet (intake 2 am) with 5 mg of prednisone,
   B) Novel "Prednisone TR" tablet, "semi-fasted" (intake 8 pm) with 5 mg of prednisone
   C) Novel "Prednisone TR" tablet, fed-state (intake 8 pm) with 5 mg of prednisone.
**Figure 3** shows *the in vivo* plasma level of prednisolone after administration of
   A) standard "Prednisone IR" tablet (intake 2 am) with 5 mg of prednisone,
   B) Novel "Prednisone TR" tablet, "semi-fasted" (intake 8 pm) with 5 mg of prednisone
   C) Novel "Prednisone TR" tablet, fed-state (intake 8 pm) with 5 mg of prednisone.

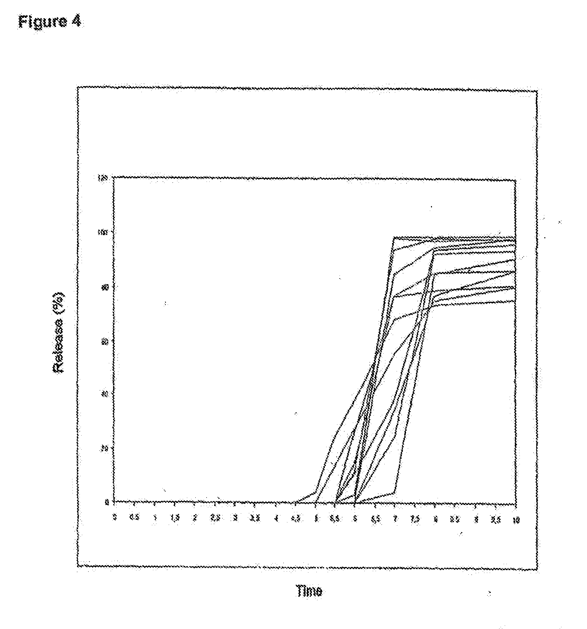

**Figure 4** shows *the in vitro* release of a "Prednisone TR" tablet containing 5 mg of prednisone with a lag phase of 6 h (500 ml of water, paddle, USP)
**Figure 5** shows an *in vivo* plasma level profile after administration of prednisone tablets.
   1) "Prednisone IR" standard tablet (intake 8 am)
   2) "Prednisone IR" standard tablet (intake 2 am)
   3) Novel "Prednisone TR" tablet with 6 h lag phase ("semi-fasted")(intake 8 pm)
   4) Novel "Prednisone TR" tablet with 6 h lag phase (fed state) (intake 8 pm)

It is possible for the site- and time-linked gastrointestinal release of a glucocorticoid to differentiate two preferred embodiments:
1) Release in the upper sections of the intestine with the following aims:
   - avoidance of instabilities of the glucocorticoid in contact with gastric juice,
   - avoidance of side effects, such as ulcers, on release of the glucocorticoid in the stomach,
   - optimal site and timing of absorption of the glucocorticoid and its entry into the plasma after release of the glucocorticoid in the upper section of the small intestinal region,
   - achievement of the systemic effect at the ideal time,
   - display of a local effect in the upper sections of the intestine.
2) Release in the lower sections of the intestine with the following aims:
   - local and targeted gastrointestinal release of glucocorticoid,
   - avoidance of side effects by glucocorticoid after (unwanted) absorption has taken place.

It is common to both embodiments to increase markedly the medicament efficacy and to reduce the side effects thereof.

A first preferred embodiment therefore provides a pharmaceutical dosage form with a release of glucocorticoid in the upper sections of the intestine within a period of 2-6 hours. A second preferred embodiment provides a pharmaceutical dosage form with a site- and time-controlled release of glucocorticoid in the lower sections of the intestine within a period of 6-10 hours after intake.

The invention described herein relates to a novel timed-release ("TR") dosage form which releases the glucocorticoid or the combination of glucocorticoids, depending on the composition, the geometry and the production conditions, at a particular site and/or at a particular time, and thus makes it possible to ensure an optimal effect with reduced side effects.

Thus, experiments have already been carried out with prednisone as model glucocorticoid ("Prednisone TR") and can, because of the comparable properties, also be applied to other glucocorticoids.

The novel "TR" dosage form described herein differs from prior art preparations. It surprisingly shows with a specific geometry of the press coating with inert adjuvants and accurately adjusted production process parameters a reproducible lag phase and subsequent rapid release (drug release phase) of the glucocorticoid or the glucocorticoid combination.

The inert coating initially prevents release of the glucocorticoid or the glucocorticoid combination over an exactly defined period, so that no absorption can occur. The water present in the gastrointestinal tract penetrates slowly in through the coating and, after a time which is previously fixed by the pressure for compression, reaches the core. The coating ingredients show neither swelling nor erosion of parts of the coating. When the core is reached, the water penetrating in is very rapidly absorbed by the hydrophilic ingredients of the core, so that the volume of the core increases greatly and, as a consequence thereof, the coating completely bursts open, and the glucocorticoid and the glucocorticoid combination respectively is released very rapidly.

A particularly advantageous embodiment of this press-coated "TR" tablet is achieved when a previously compressed core tablet is subsequently compressed with a multilayer tablet press to a press-coated tablet.

The tablet coating typically consists of the following materials in order to achieve a delayed release profile:
- polymer or copolymer of acrylic acid, methacrylic acid etc. (e.g. Eudragits or Carbopol),
- cellulose derivatives such as hydroxypropylmethylcellulose, hydroxypropylcellulose, carboxymethylcellulose, ethylcellulose, cellulose acetate,
- polyvinyl alcohol,
- polyethylene glycol,
- salts of higher fatty acids, esters of monohydric or polyhydric alcohols with short-medium- or long-chain, saturated or unsaturated fatty acids. Specifically, stearic acid triglycerides (e.g. Dynersan) or glycerol behenate (e.g. Compritol) are used.

In addition, further adjuvants should also be added to these materials so that the tablet coating can be compressed. Typically used here are fillers such as lactose, various starches, celluloses and calcium hydrogen phosphate. The glidant used is normally magnesium stearate, and in exceptional cases also talc and glycerol behenate. A plasticizer is often also added to the coating material, preferably from the group of polyethylene glycol, dibutyl phthalate, Diethyl citrate or triacetin.

In order to achieve an optimal release profile, the tablet core must also fulfill certain tasks and exhibit certain properties. Thus, after the lag phase has elapsed, a rapid release profile is achieved if typical disintegrants are added to the inner core, which are derived for example from the group of the following substances: cellulose derivatives, starch derivatives, cross linked polyvinylpyrrolidone. The use of a blowing agent, for example resulting from a combination of a weak acid and a carbonate or bicarbonate, may also promote rapid release. The tablet core typically consists additionally of matrix or filling ingredients (e.g. lactose, cellulose derivatives, calcium hydrogen phosphate or other substances known from the literature) and lubricant or glidant (usually magnesium stearate, in exceptional cases also talc and glycerol behenate).

The size of the core tablet preferably should not exceed 6 mm (preferably 5 mm) in diameter, because otherwise the press-coated tablet becomes too large for convenient ingestion. As a result thereof, the dosages of the glucocorticoid are in the range from 0.1 to 50 mg, very particularly between 1 and 20 mg.

The *in vitro* release profile of the "TR" dosage form according to the invention is preferably such that less than 5% of the glucocorticoid is released during the lag phase. After the release phase has started, preferably ≥80%, particularly preferably ≥90%, of the glucocorticoid is released within one hour. The *in vitro* release is preferably determined using the USP paddle dissolution model in water.

The employed glucocorticoids are from the following group and all show comparable physicochemical properties; cortisone, hydrocortisone, prednisone, prednisolone, methylprednisolone, budesonide, dexamethasone, fludrocortisone, fluocortolone, cloprednole, deflazacort, triamcinolone, and the corresponding salts and esters thereof. This applies in particular to prednisone, prednisolone, methylprednisolone, budesonide, dexamethasone, fluocortolone, cloprednole, and deflazacort and the corresponding salts and esters thereof.

In the present case of the "TR" tablet, the following combination of core materials and coating materials has proved to be particularly suitable for achieving a time- and site-controlled release with exclusion of pH and food influences:
The coating preferably comprises:
   - hydrophobic, waxy substances with a HLB value of less than about 5, preferably around 2. Carnauba wax, paraffins, cetyl ester waxes are preferably employed therefore. Glycerol behenate has proved to be particularly suitable. The use of about 20-60%, in particular about 30-50%, in the coating has proved to be very advantageous;
   - non-fatty, hydrophobic filling materials such as calcium phosphate salts, e.g. dibasic calcium phosphate. The use of about 25-75% of these filling materials, in particular of about 40-60%, in the coating has proved to be very advantageous here;
   - in addition, the tablet coating preferably also consists of binders, e.g. polyvinylpyrrolidone (PVP), typically in concentrations of about 4-12%, specifically about 7-10%, and glidants such as magnesium stearate, in concentrations of about 0.1-2%, in the specific case of about 0.5-1.5%. Colloidal silicon dioxide can for example be used as flow regulator, normally in concentrations of about 0.25-1%. In addition, to distinguish different dosages, a colorant can be added to the tablet coating, preferably an iron oxide pigment in concentrations of about 0.001-1%.

The core tablet comprises:
- glucocorticoids, selected from prednisone, prednisolone, methylprednisolone, budesonide, dexamethasone, fludrocortisone, fluocortolone, cloprednole, deflazacort, and triamcinolone, and the corresponding salts and esters thereof. The dosages of the glucocorticoid are in the region of about 0.1-50 mg, very especially between about 1 and 20 mg;
- in addition, the core tablet preferably comprises a filler such as, for example, lactose, starch derivatives or cellulose derivatives. Lactose is preferably employed. The filler is typically present in concentrations of about 50-90%, specifically of about 60-80%. A disintegrant is additionally present and is typically cross linked PVP or sodium carboxymethylcellulose, typically in concentrations of about 10-20%. It is additionally possible for a binder, e.g. PVP, to be present, typically in concentrations of about 2-10%, specifically of about 5.5-9%, and a lubricant such as magnesium stearate, in concentrations of about 0.1-2%, in the specific case of about 0.5-1.5%. Colloidal silicon dioxide is normally used as flow regulator, normally in concentrations of about 0.25-1 %. It is additionally possible, for visually distinguishing the core from the coating, to add a colorant, preferably an iron oxide pigment in concentrations of about 0.01-1%.

The pharmaceutical dosage form according to the invention is preferably distinguished by the *in vitro* release and the *in vivo* release (on oral intake) of the glucocorticoid not differing by more than about one hour, particularly preferably not more than about 30 minutes. It is further preferred for *the in vitro* release to be substantially independent of the pH of the release medium or/and of additions in the release medium which simulate high-fat and low-fat food, i.e. to vary by preferably not more than about ±20%. It is further preferred for *the in vivo* release to be substantially independent of food intake, with the time to reach the maximum plasma concentration (tₘₐₓ) varying by not more than about ±20%. The plasma level reached *on in vivo* release is preferably independent of the gastrointestinal pH and of food intake.

On *in vivo* release in the upper sections of the intestine, preferably equivalent parameters, in particular a maximum plasma level (Cₘₐₓ) reached or/and an area reached under the plasma curve (AUC), as for a rapid- release dosage form are achieved. It is particularly preferred for a Cₘₐₓ of at least about 70%, preferably of at least about 80%, of the Cₘₐₓ of a rapid-release dosage form, and an AUC which does not vary by more than about ±25%, to be achieved. On release in lower sections of the intestine, *the in vivo* plasma levels reached are much lower, this likewise being substantially independent of the gastrointestinal pH and of food intake. The latter embodiment of the invention is thus particularly suitable for the treatment of local inflammatory bowel disease such as Crohn's disease or ulcerative colitis, where a systemic effect is not desired. The first-mentioned embodiment, with which absorption takes place in the upper sections of the intestine, is by contrast suitable in particular for the treatment of inflammatory diseases of the joints, associated with pain, such as, for example, rheumatoid arthritis, allergies and nocturnal severe asthmatic attacks, where a systemic effect is desired.

The process for producing the tablet takes place under usual conditions of the pharmaceutical industry. Thus, standard technologies are used in the production of the core tablet, such as weighing, sieving, mixing, aqueous granulation in a high-speed mixer, fluidized-bed drying of the granules, mixing and compression. Comparable methods are employed to produce the coating, namely weighing, sieving, mixing, aqueous granulation in a high-speed mixer, fluidized-bed drying of the granules, mixing and compression to press-coated tablets.

The geometry of the press-coated tablet has, in addition to the composition, a very great importance. It can be achieved only using a tablet machine for producing press-coated tablets; spray coatings are unsuitable.

The ratio of the thickness of the press-coating on the sides of the tablets to the upper side or lower side is preferably about 2.2-2.6 mm (for the side edges): about 1.2-1.6 mm for the upper side of the tablet and about 1.0-1.4 mm (for the lower side of the tablet), particularly preferably about 2.35-2.45 mm:about 1.35-1.45 mm (upper side of the tablet) and about 1.15-1.25 mm (lower side). This geometry results in the tablet remaining sufficiently small to avoid problems with swallowing.

Tablets with a hardness of about 60-90 N, measured as specified in the European Pharmacopoeia 4, 2.9.8, are thus achieved.

The timed-release ("TR") of glucocorticoid can be controlled by setting the compressive forces during the application of the coating to the tablet core. Thus, the compressive forces used for release in the upper sections of the intestine are preferably up to about 600 kg, particularly preferably about 250-600 kg, whereas the compressive forces used for release of the glucocorticoid in the lower sections of the intestine are preferably above about 600 kg, particularly preferably about 600-800 kg.

The pharmaceutical dosage form is particularly preferably in the form of a tablet, but it is also possible to produce the dosage form as capsule.

The present invention is further illustrated by the following examples.

### Examples

### Example 1: Formulas

Core tablet consisting of:

| | |
|---|---|
| Glucocorticoid | 1 mg |
| Lactose | 42.80 mg |
| Povidone | 4 mg |
| Carboxymethylcellulose, Na | 11 mg |
| Iron oxide, red | 0.3 mg |
| Magnesium stearate | 0.6 mg |
| Silicon dioxide | 0.3 mg |

or

| | |
|---|---|
| Glucocorticoid | 5 mg |
| Lactose | 38.80 mg |
| Povidone | 4 mg |
| Carboxymethylcellulose, Na | 11 mg |
| Iron oxide, red | 0.3 mg |
| Magnesium stearate | 0.6 mg |
| Silicon dioxide | 0.3 mg |

or

| | |
|---|---|
| Glucocorticoid | 10 mg |
| Lactose | 33.80 mg |
| Povidone | 4 mg |
| Carboxymethylcellulose, Na | 11 mg |
| Iron oxide, red | 0.3 mg |
| Magnesium stearate | 0.6 mg |
| Silicon dioxide | 0.3 mg |

Coating consisting of:

| | |
|---|---|
| Calcium phosphate | 50% |
| Glycerol behenate | 40% |
| Povidone | 8.4% |
| Iron oxide, yellow | 0.1% |
| Magnesium stearate | 1.0% |
| Silicon dioxide | 0.5% |

Glucocorticoid is from the group; cortisone, hydrocortisone, prednisone, prednisolone, methylprednisolone, budesonide, dexamethasone, fludrocortisone, fluocortolone, cloprednole, deflazacort, triamcinolone and the corresponding salts and esters thereof.
The composition of the tablets ensures that the influences of food, pH and motility of the gastrointestinal tract have no influence, and the glucocorticoid escapes very rapidly from the tablet after completion of the lag phase.

### Example 2: Production process and in vitro release

With a fixed tablet geometry, the lag phase of glucocorticoid release is determined exclusively by the variably adjustable compressive force. Prednisone was used as a glucocorticoid in this case.

Thus, an average pressure of 400 kg for compression leads for example to a lag phase of 4 hours. Table 1 summarizes the lag phases as a function of the compressive force:

**Table 1 : Dependence of the lag phase [h] on the average compressive force [kg]**

| **Compressive force [kg]** | **lag phase [h]** |
|---|---|
| 300 | 3 |
| 340 | 3.5 |
| 400 | 3.9 |
| 460 | 4.5 |
| 580 | 5 |

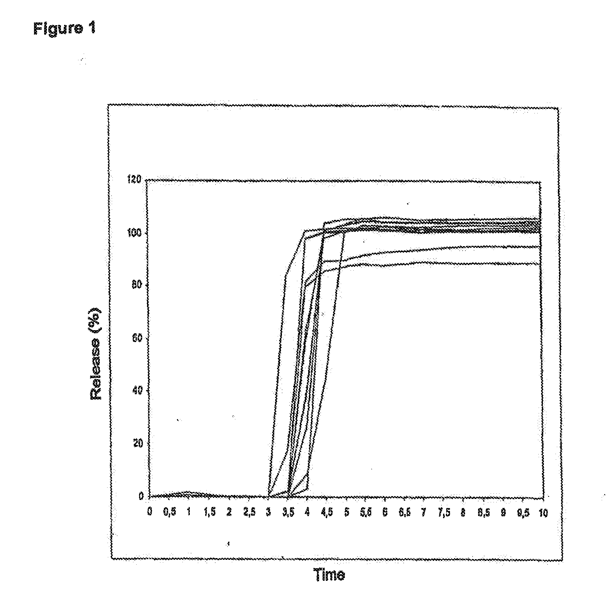

The lag phase is determined by means of the USP paddle dissolution model with 100 rpm in water at a temperature of 37°C. Figure 1 shows typical release behaviour (batch G360).

Surprisingly, the lag phases and drug release phases in hours are comparable in different release media for this formulation, with fixed geometry and identical compressive force. This is evident from Table 2 (batch: G360).

**Table 2: Lag phases and drug release phases [h] of the novel "Prednisone TR" tablet with the active ingredient prednisone in different release media, in vitro dissolution release, 500 ml, paddle, USP**

| **Medium** | **Average lag phase [h]** | **Average drug release phase [h]** |
|---|---|---|
| Water | 4.1 | 0.7 |
| pH 1.2 | 3.6 | 0.8 |
| pH 4.5 | 3.8 | 0.9 |
| pH 6.8 | 4.0 | 0.9 |
| FaSSIF | 4.2 | 0.8 |
| FeSSIF | 4.1 | 0.9 |

This surprising finding is very important because the aim which it is intended to pursue is to achieve site- and time-controlled release without the influence of food.

Further experiments to correlate the compressive force with the lag phase were undertaken with respect to 1 mg and 5 mg tablets containing prednisone as the glucocorticoid. The results are summarized below:

| **Compressive Force** | **Average lag phase 1 mg tablet** | **Average lag phase 5 mg tablet** |
|---|---|---|
| 150 kg | 2.2 | 2.2 |
| 200 kg | 2.4 | 2.7 |
| 400 kg | 3.4 | 3.9 |
| 600 kg | 4.2 | 5.1 |
| 800 kg | 4.8 | 5.6 |
| 1200 kg | 6.0 | |

Surprisingly, there are some differences in the required compression forces between TR tablets of different strengths. Therefore, testing of the *in-vitro* characteristics of each batch in a dissolution apparatus is preferred to confirm release of the glucocorticoid at a specific lag-time. This can easily be monitored by a color change of the dissolution medium. The color is released from the colored core tablets.

### Example 3: In vivo release

It was surprisingly possible to confirm *in vivo* exactly the delay, measured *in vitro,* of prednisone release.

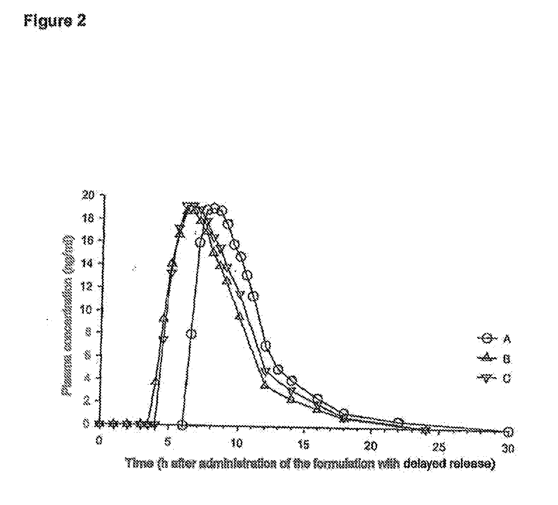

It was possible to show in a pharmacokinetics study that with a delay of 4 hours in glucocorticoid release *in vitro,* the delay *in vivo* is exactly the same, and there is subsequently a very rapid rise in level. The resulting plasma levels of prednisone after administration of the novel "Prednisone TR" tablet are depicted in Figure 2. They agree very well in terms of time with the *in vitro* release profile. It was additionally found that simultaneous intake of food evidently likewise has no influence *in vivo,* and comparable plasma levels are found as in the "semi-fasted" state. This is surprising because food normally influences the motility of the gastrointestinal tract, the pH, the luminal metabolism, and normally interacts with the dose form. The Guidance for Industry "Food Effect Bioavailability and Fed Bioequivalence Studies" of the US FDA, Department of Health of December 2002 mentions that a difference in reaching the tₘₐₓ ought to be of no clinical relevance.

It is therefore very gratifying that the lag phase for the present "Prednisone TR" tablet *in vitro* is 4 hours and this is also found *in vivo* with and without food. In addition, food evidently has no influence on the maximum plasma levels (Cₘₐₓ) reached and the areas reached under the plasma curve (AUC) either. The time until the maximum plasma concentration (tₘₐₓ) is reached is likewise independent of the intake of food.

The difference in tₘₐₓ between the tablet in the semi-fasted state compared with simultaneous food intake is a maximum of ±20% and is thus clinically insignificant.

To demonstrate the influence of food on the release of the glucocorticoid from the novel "Timed-Release" dosage form, the applicant has carried out a pharmacokinetics study on 27 subjects. Three arms were compared: administration in the evening (8 pm) of the novel "Prednisone TR" tablet with standardized supper (fed state), administration in the evening (8 pm) of the novel "Prednisone TR" tablet with light supper around 17.30 h (semi-fasted), administration at night (2 am) of a standard Prednisone Immediate Release tablet (Decortin, Merck, Germany). The study was carried out randomized, in cross-over design as single dose administration and thus complies with the usual regulatory requirements.

The aim of the kinetics study was to achieve comparable plasma level profiles in relation to Cₘₐₓ and AUC for the novel tablet "Prednisone TR" "semi-fasted" compared with "fed state" in relation to a standard "Prednisone IR" tablet (with rapid release of active ingredient). The novel tablet described herein with the glucocorticoid prednisone showed that comparable plasma level profiles can be achieved.

The plasma samples were taken at intervals of 0.5 and later of 1 hour.

The prednisone plasma levels found are depicted graphically in Figure 2, and the principal pharmacokinetic characteristics are summarized in Table 3.

**Table 3: Pharmacokinetic parameters for prednisone after administration of a single dose of 5 mg of prednisone as "Prednisone IR" or "Prednisone TR" tablet in 27 healthy male volunteers**

| **Parameter** | **Prednisone IR a2am** | **Prednisone TR; semi-fasted at 8 pm** | **Predisone TR; fed state at 8pm** | **p*** |
|---|---|---|---|---|
| Cₘₐₓ | 20.9 | 20.3 | 22.0 | 0.54 |
| (ng/mL) | (19.2-22.7) | (18.6-22.1) | (20.1-23.9) | |
| tₘₐₓ (h) | 2 (1.0-4.0) | 6.0 (4.5-10.0) | 6.5 (4.5-9.0) | <0.0001 |
| t_{lag} (h) | 0.0 | 3.5 (2.0-5.5) | 4.0 (3.5-5.0) | <0.0001 |
| | (0.0-0.5) | | | |
| AUC₀₋ₜ | 107 | 108 | 121 | 0.16 |
| (h.ng/mL) | (98.8-116) | (99.1-117) | (111-132) | |
| AUC_{0∼∞} | 109 | 110 | 123 | 0.15 |
| (h.ng/mL) | (101-118) | (102-119) | (114-134) | |
| t_{1/2} (h) | 2.57 | 2.41 | 2.41 | 0.002 |
| | (2.51-2.63) | (2.36-2.47) | (2.36-2.47) | |

| | | | | |
|---|---|---|---|---|
| tₘₐₓ and t_{tag} values are means (range). The other values are geometric means (90% Cl) obtained from ANOVA. *: probability associated with the hypothesis, that there is no difference between the formulations (ANOVA, except for tₘₐₓ and t_{tag}: Friedman test) | | | | |

It was also possible to confirm these results for prednisolone, a metabolite of prednisone, after administration of the novel "Prednisone TR" tablet.

Thus, it was also possible to show for prednisolone a comparability between Cₘₐₓ and AUC of the novel "Prednisone TR" tablet "semi-fasted" with "fed state". The plasma level profile of the metabolite prednisolone is therefore also independent of food intake.

The plasma samples for determining prednisolone were taken at intervals of 0.5 and later of 1 hour.

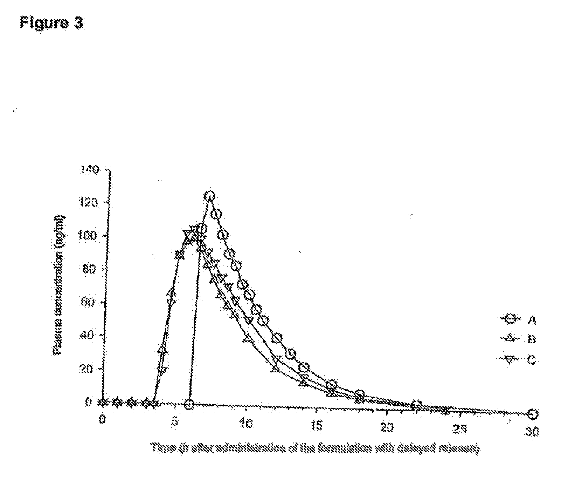

The plasma levels found for prednisolone are depicted graphically in Figure 3, and the principal pharmacokinetic characteristics are summarized in Table 4.

**Table 4: Pharmacokinetic parameters for prednisolone after administration of a single dose of 5 mg of prednisone as "Prednisone IR" or "Prednisone TR" tablet in 27 healthy male volunteers**

| **Parameter** | **Prednisone IR at 2 am** | **Prednisone TR; semi-fased at 8 pm** | **Prednisone TR; fed state at 8 pm** | **p*** |
|---|---|---|---|---|
| Cₘₐₓ | 135 | 113 | 132 | 0.036 |
| (ng/mL) | (124-147) | (104-123) | (121-143) | |
| tₘₐₓ (h) | 1.0 | 5.0 (4.0-9.0) | 5.5 (4.5-9.0) | <0.0001 |
| | (0.5-3.0) | | | |
| t_{tag} (h) | 0.0 | 3.5 (2.0-5.5) | 3.5 (3.0-5.0) | <0.0001 |
| | (0.0-0.5) | | | |
| AUC₀₋ₜ | 614 | 561 | 647 | 0.081 |
| (h.ng/mL) | (571-661) | (520-605) | (599-698) | |
| AUC_{0∼∞} | 624 | 573 | 658 | 0.0076 |
| (h.ng/mL) | (582-670) | (533-616) | (612-707) | |
| t_{1/2} (h) | 2.66 | 2.66 | 2.71 | 0.11 |
| | (2.63-2.70) | (2.62-2.69) | (2.68-2.75) | |

| | | | | |
|---|---|---|---|---|
| tₘₐₓ and t_{tag} values are means (range). The other values are geometric means (90% Cl) obtained from ANOVA. *: probability associated with the hypothesis, that there is no difference between the formulations (ANOVA, except for tₘₐₓ and t_{tag} Friedman test) | | | | |

Typical achieved Cₘₐₓ values for 5 mg prednisone tablets after ingestion will be in the range of from about 15 to about 25 ng/ml, and the AUC of prednisone is from about 75 to about 150 h*ng/mL The achieved Cₘₐₓ values for the prednisolone metabolite will be in the range of from about 100 to about 160 ng/ml, and the AUC of prednisolone is from about 500 to about 700 h*ng/ml.

It should additionally be mentioned that the coefficients of variation for Cₘₐₓ, tₘₐₓ and AUC for prednisone after administration of the standard tablet "Prednisone IR" (at 2 am) and of the novel tablet "Prednisone TR" (at 8 pm) with and without food are approximately comparable. This has not previously been described for a tablet with modified release of glucocorticoid. Table 5 summarizes the coefficients of variation for prednisone.

**Table 5: Coefficients of variation for Cₘₐₓ tₘₐₓ, AUC for prednisone plasma levels after administration of a standard tablet "Prednisone IR", of the novel tablet "Prednisone TR" "semi-fasted" and in "fed state"**

| | | **Prednisone IR at 2 am** | **Prednisone TR; semi-fasted at 8 pm** | **Prednisone TR; fed state at 8 pm** |
|---|---|---|---|---|
| **N** | | 26 | 26 | 26 |
| **Cₘₐₓ (ng/ml)** | **Average** | 21.1 | 21.4 | 22.2 |
| | **SD** | 3.56 | 5.65 | 3.66 |
| | **Median** | 20.8 | 21.4 | 22.2 |
| | **CV** | 16.9 | 26.4 | 16.4 |
| **tₘₐₓ (h)** | **Average** | 2.06 | 6.21 | 6.5 |
| | **SD** | 0.68 | 1.22 | 1.11 |
| | **Median** | 2 | 6 | 6.5 |
| | **CV** | 33.1 | 19.6 | 17.1 |
| **AUC_{0∼∞} (ng/ml*h)** | **Average** | 111 | 116 | 126 |
| | **SD** | 17.5 | 31 | 24.3 |
| | **Median** | 106 | 122 | 130 |
| | **CV (%)** | 15.8 | 26.6 | 19.2 |

The coefficients of variation of the pharmacokinetic parameters for the metabolite prednisolone likewise differ negligibly when the standard tablet is compared with the novel tablet.

**Table 6: Coefficients of variation for Cₘₐₓ, tₘₐₓ, AUC for prednisolone plasma levels after administration of a standard tablet "Prednisone IR", of the novel tablet "Prednisone TR" "semi-fasted" and in fed state**

| | | **Prednisone IR at 2 am** | **Prednisone TR; semi-fasted at 8 pm** | **Prednisone TR; fed state at 8 pm** |
|---|---|---|---|---|
| **N** | | 26 | 26 | 26 |
| **Cₘₐₓ (ng/ml)** | **Average** | 138 | 121 | 135 |
| | **SD** | 22.9 | 32.3 | 24.5 |
| | **Median** | 140 | 130 | 135 |
| | **CV** | 16.6 | 26.8 | 18.2 |
| **tₘₐₓ (h)** | **Average** | 1.12 | 5.58 | 5.81 |
| | **SD** | 0.67 | 1.2 | 1.16 |
| | **Median** | 1 | 4 | 5.5 |
| | **CV** | 59.3 | 21.5 | 19.9 |
| **AUC_{0∼∞} (ng/ml*h)** | **Average** | 638 | 611 | 680 |
| | **SD** | 112 | 178 | 142 |
| | **Median** | 646 | 677 | 713 |
| | **CV (%)** | 17.7 | 29.2 | 20.9 |

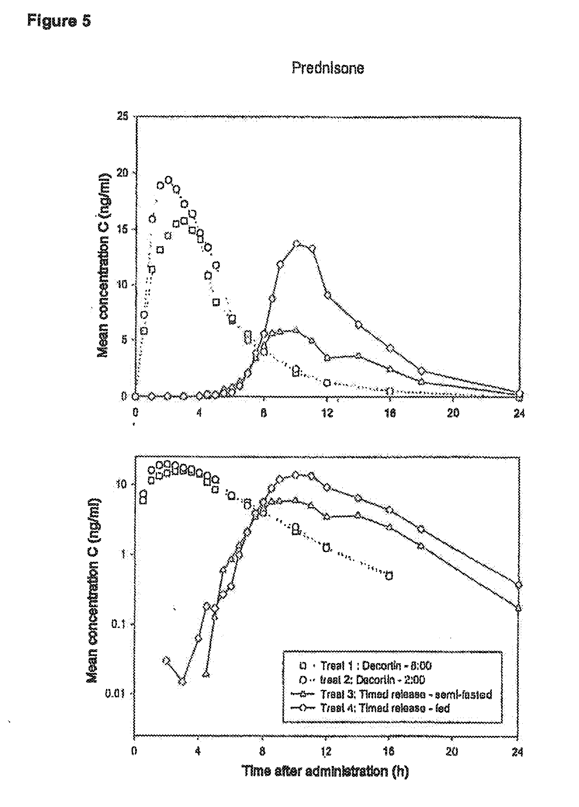

The situation is quite different when a tablet with a longer lag phase (6 hours *in vitro)* is administered. It is true that release after 6 hours is in this case also found *in vitro.* However, at the same time, the absorption is greatly reduced because the release obviously takes place in lower sections of the intestine, and absorption now takes place only to a smaller extent. This was shown in a second pharmacokinetics study. Figure 5 shows a novel "Prednisone TR" tablet with a lag phase of 6 hours, which can be produced by means of a higher pressure for compression.

Typical achieved Cₘₐₓ values for such 5 mg prednisone tablets after ingestion will be in the range of less than 15 ng/ml, and the AUC of prednisone is less than 75 h*ng/mL. The achieved Cₘₐₓ values for the prednisolone metabolite will be in the range of less than 100 ng/ml, and the AUC of prednisolone is less than 500 h*ng/mL

Very interesting novel therapeutic approaches derive therefrom, and this invention relates thereto. Thus, the composition of the tablet, its specific geometry and a compressive force which can be adjusted variably make it possible for the coating of the tablet to release the glucocorticoid very rapidly from the core tablet after an exactly fixed time. This is very advantageous because the site of release can also be fixed accurately via this precise presetting.

It is possible with a site of release on the one hand to treat local disorders of the gastrointestinal tract locally. For example, ulcerative colitis, an inflammatory disorder of the bowel, may affect different sections of the intestine. This novel timed-release ("TR") tablet is very advantageous especially for disorders of lower sections of the intestine, because there is now mainly local release of the glucocorticoid, but absorption thereof is only negligible or very limited. It is possible thereby to avoid effects which normally occur after uptake of the glucocorticoid into the blood.

However, with a precise controlled glucocorticoid release after an exactly defined lag phase it is also possible to achieve the plasma level profiles which correspond to those after administration of a rapid-release tablet. However, the precondition for this is that the coating of the novel timed- release ("TR") tablet exposes the glucocorticoid-containing core after less than 6 hours, and the glucocorticoid can then be very rapidly dissolved and absorbed. One application thereof is, for example, the administration of glucocorticoids for the treatment of inflammatory disorders to the joints, where pro-inflammatory cytokines are released in the early hours of the morning and are thought to be responsible for the pain in the morning and the stiffness of fingers in the morning. It is now possible through the novel tablet, with intake at 10 pm, to enable release at 2 am, and thus to ensure the optimal effect, described by Arvidson et al. (Annals of Rheumatic Diseases 1997; 56:27-31) of the glucocorticoid on rheumatoid arthritis and, in addition, to contribute to a crucial increase in patient compliance. Consequently, the tablets of the present invention may be ingested once daily at bed-time, for example between about 8 pm and about 12 am, and more preferably between about 9 pm and about 11 pm.

The present invention also provides a method for producing a tablet that releases a glucocorticoid at a predetermined variable location in the Gl tract, said method comprising:
determining the location in the Gl tract at which it is desired to deliver the glucocorticoid;
forming a coated tablet having a core comprising the glucocorticoid and a swellable adjuvant, and an inert outer coating;
compressing the coating of said tablet at a pressure chosen to result in the release of the glucocorticoid at said predetermined position; and
testing the *in vitro* release characteristics in a dissolution apparatus in order to confirm release of the glucocorticoid at a specific lag time. The *in vitro* release characteristics can then be correlated to the suitable *in vivo* release lag time.
In a preferred embodiment, the tablet core comprises a coloring material, and *the in vitro* release of the glucocorticoid is determined by a color change. The dissolution apparatus may be any standard apparatus in the industry, and preferably is in accordance with USP XXVIII.

## Claims

1. A method of producing a tablet which releases a glucocorticoid active ingredient at a predetermined variable location in the GI tract, said method comprising
determining the location in the GI tract at which it is desired to deliver the glucocorticoid;
forming a coated tablet having a core comprising the glucocorticoid and a swellable adjuvant, and an inert outer coating which is non-swelling and non-eroding; and
compressing the coating of said tablet at a pressure chosen to result in the release of the glucocorticoid at said predetermined position.

2. The method of claim 1, wherein the glucocorticoid is rapidly released when the core is contacted with gastrointestinal fluids; and wherein said coating is capable of preventing substantial release of the glucocorticoid for a defined time period following ingestion of the dosage form.

3. The method of claim 1 or claim 2, wherein the core further comprises a disintegrant.

4. The method of any one of claims 1 to 3, wherein the glucocorticoid ingredient is released in the upper sections of the intestine within a period of about 2 to about 6 hours after ingestion, wherein a systemic effect occurs *on in vivo* release of the glucocorticoid in the upper sections of the intestine, and wherein the coating is produced by compressive forces of up to about 600 kg.

5. The method of any one of claims 1 to 3, wherein the glucocorticoid is released in the lower sections of the intestine within a period of about 6 to about 10 hours after ingestion, wherein a substantially local effect occurs *on in vivo* release of the glucocorticoid in the lower sections of the intestine, and wherein the coating is produced by compressive forces above about 600 kg.

6. The method of claim 1, wherein
(i) *the in vitro* release and the *in vivo* release of the glucocorticoid do not differ by more than about 1 hour,
(ii) the *in vitro* release of the glucocorticoid is substantially independent of the pH of the release medium or/and of additions in the release medium which simulate high-fat and low-fat food, or
(iii) *the in vivo* release is substantially independent of food intake.

7. The method of claim 4, wherein the plasma level reached *on in vivo* release of the glucocorticoid is independent of the gastrointestinal pH and of food intake.

8. The method of claim 4, wherein *the in vivo* biopharmaceutical/pharmacokinetic profile of the glucocorticoid active ingredient or its active metabolite is at least substantially identical to that of an immediate release tablet regarding Cₘₐₓ and/or AUC.

9. The method of claim 8 wherein
(i) the tablet comprises about 5 mg of prednisone, and wherein the achieved Cₘₐₓ of prednisone after ingestion is from about 15 to about 25 ng/mL and/or the AUC of prednisone is from about 75 to about 150 h*ng/mL, or
(ii) the tablet comprises about 5 mg of prednisolone, and/or wherein the achieved Cₘₐₓ of the prednisolone active metabolite after ingestion is from about 100 to about 160 ng/mL and the AUC of the prednisolonem active metabolite is from about 500 to about 700 h*ng/mL.

10. The method of claim 1, wherein the achieved tₘₐₓ of the glucocorticoid is from about 2 to about 8 hours after ingestion.

11. The method of claim 1, wherein the core comprises
- the glucocorticoid;
- from about 50% to about 90% of a filler;
- from about 10% to about 20% of a disintegrant,
- from about 2% to about 10% of a binder;
- from about 0.1 % to about 2% of a glidant;
- from about 0.25% to about 1% of a flow regulator; and
- from 0% to about 1 % of a pigment;
all based on the total weight of the core.

12. The method of claim 11, wherein the filler comprises lactose; the disintegrant comprises crosslinked polyvinylpyrrolidone, sodium carboxymethylcellulose, or mixtures thereof; the binder comprises uncrosslinked polyvinylpyrrolidone; the lubricant comprises magnesium stearate; the flow regulator comprises colloidal silicon dioxide; and the pigment comprises iron oxide.

13. The method of claim 1, wherein the coating comprises
- from about 20% to about 60% of a hydrophobic waxy substance;
- from about 25% to about 75% of a non-fatty hydrophobic filling material;
- from about 4% to about 12% of a binder;
- from about 0.1 % to about 2% of a glidant;
- from about 0.25% to about 1% of a flow regulator; and
- from about 0% to about 1 % of a pigment;
all based on the total weight of the coating.

14. The method of claim 13, wherein the hydrophobic waxy substance comprises glycerol behenate and the non-fatty hydrophobic filler comprises calcium phosphate, particularly dibasic calcium phosphate or basic calcium phosphate.

15. The method of claim 1, wherein the glucocorticoid comprises more than one glucocorticoid.

16. The method of claim 1, wherein the glucocorticoid is selected from the group consisting of cortisone, hydrocortisone, prednisone, prednisolone, methylprednisolone, budesonide, dexamethasone, fludrocortisone, fluocortolone, cloprednole, deflazacort, triamcinolone and pharmaceutically acceptable salts and esters thereof, and mixtures thereof.

17. The method of claim 1, wherein the glucocorticoid is selected from the group consisting of prednisone, prednisolone, methylprednisolone and budesonide.

18. The method of claim 1, wherein the amount of glucocorticoid is from about 0.1 mg to about 20 mg.

19. The method of claim 4, wherein the tablet is ingested once daily at bedtime, particularly between about 8 pm and about 12 am and more particularly between about 9 pm and about 11 pm.

20. The method of claim 4, wherein the glucocorticoid is effective for the treatment of inflammatory disorders of the joints, pain, allergies or nocturnal severe asthmatic attacks.

21. The method of claim 1, wherein the glucocorticoid is effective for the treatment of a local inflammatory bowel disorder, particularly selected from the group consisting of Crohn's disease and ulcerative colitis.

22. The method of claim 4, wherein the glucocorticoid is effective for the treatment of a local inflammatory bowel disorder in the upper sections of the intestine, wherein the disorder is particularly selected from the group consisting of Crohn's disease and ulcerative colitis.

23. The method of claim 5 wherein the glucocorticoid is effective for the treatment of a local inflammatory bowel disorder in the lower sections of the intestine, wherein the disorder is particularly selected from the group consisting of Crohn's disease and ulcerative colitis.

24. The method of claim 1, further comprising testing the in vitro release characteristics in a dissolution apparatus in order to confirm release of the glucocorticoid at a specific lag time.

25. The method of claim 24, wherein the core comprises a coloring material, and the release of the glucocorticoid is determined by a color change.

26. A coated tablet having a core of a glucocorticoid active ingredient and a swellable adjuvant and an inert outer coating which is non-swelling and non-eroding, capable of releasing the glucocorticoid at a predetermined variable location of the GI tract, the coating being compressed to a degree which results in the release of the glucocorticoid at said predetermined location.

27. The tablet of claim 26, wherein the glucocorticoid is rapidly released when the core is contacted with gastrointestinal fluids; and wherein said coating is capable of preventing substantial release of the glucocorticoid for a defined time period following ingestion of the dosage form.

28. The tablet of claim 26 or claim 27, wherein the core further comprises a disintegrant.

29. The tablet of claim 26, wherein the glucocorticoid is released in the upper sections of the intestine within a period of from about 2 to about 6 hours after ingestion, wherein a systemic effect occurs *on in vivo* release of the glucocorticoid in the upper sections of the intestine and wherein the coating is produced by compressive forces of up to about 600 kg.

30. The tablet of claim 26, wherein the glucocorticoid is released in the lower sections of the intestine within a period of from about 6 to about 10 hours after ingestion, wherein a substantially local effect occurs on *in vivo* release in the lower sections of the intestine, and wherein the coating is produced by compressive forces above about 600 kg.

31. The tablet of claim 26, wherein the glucocorticoid comprises more than one glucocorticoid.

32. The tablet of claim 27, wherein the glucocorticoid is selected from the group consisting of cortisone, hydrocortisone, prednisone, prednisolone, methylprednisolone, budesonide, dexamethasone, fludrocortisone, fluocortolone, cloprednole, deflazacort, triamcinolone and pharmaceutically acceptable salts and esters thereof, and mixtures thereof.

33. The tablet of claim 27, wherein the glucocorticoid is selected from the group consisting of prednisone, prednisolone, methylprednisolone and budesonide.

34. The tablet of claim 29, wherein the glucocorticoid is effective for the treatment of inflammatory disorders of the joints, pain, allergies or nocturnal severe asthmatic attacks.

35. The tablet of claim 27, wherein the glucocorticoid is effective for the treatment of a local inflammatory bowel disorder, particularly selected from the group consisting of Crohn's disease and ulcerative colitis.

36. The tablet of claim 29, wherein the glucocorticoid is effective for the treatment of a local inflammatory bowel disorder in the upper sections of the intestine, wherein the disorder is particularly selected from the group consisting of Crohn's disease and ulcerative colitis.

37. The tablet of claim 30, wherein the glucocorticoid is effective for the treatment of a local inflammatory bowel disorder in the lower sections of the intestine, wherein the disorder is particularly selected from the group consisting of Crohn's disease and ulcerative colitis.

38. The tablet of claim 37, wherein a substantially local effect occurs on *in vivo* release in the lower sections of the intestine, and wherein a systemic effect is not exhibited.

39. Use of a coated tablet according to any of claims 26 to 38 for the treatment of an inflammatory disorder.

40. The use of claim 39, wherein the inflammatory disorder is a local inflammatory bowel disorder, particularly selected from the group consisting of Crohn's disease and ulcerative colitis.

41. The use of claim 39, wherein the inflammatory disorder is an inflammatory disorder of the joints, pain, allergies or nocturnal severe asthmatic attacks.
